(19) Europäisches Patentamt
European Patent Office
Office européen des brevets

(11) Veröffentlichungsnummer: **0 292 816 B1**

(12) # EUROPÄISCHE PATENTSCHRIFT

(45) Veröffentlichungstag der Patentschrift: **10.07.91 Patentblatt 91/28**

(51) Int. Cl.[5]: **C07C 253/04,** C07C 255/35

(21) Anmeldenummer: **88107770.5**

(22) Anmeldetag: **14.05.88**

(54) **Verfahren zur Herstellung von m-Triflourmethylphenylacetonitril.**

Die Akte enthält technische Angaben, die nach dem Eingang der Anmeldung eingereicht wurden und die nicht in dieser Patentschrift enthalten sind.

(30) Priorität: **23.05.87 DE 3717434**

(43) Veröffentlichungstag der Anmeldung: **30.11.88 Patentblatt 88/48**

(45) Bekanntmachung des Hinweises auf die Patenterteilung: **10.07.91 Patentblatt 91/28**

(84) Benannte Vertragsstaaten: **BE CH DE FR GB IT LI**

(56) Entgegenhaltungen:
**EP-A- 0 034 763**
**DE-A- 2 854 210**
**FR-A- 1 033 516**

(56) Entgegenhaltungen:
**US-A- 4 144 265**
**Kirk-Othmer "Encyclopedia of Chemical Technology", 3e Ausgabe, Band 10 (1980) S.918-921; Aromatic Fluorine Compounds, A.E. Pavlath and A.J. Leffler, S.54-55**

(73) Patentinhaber: **Degussa Aktiengesellschaft**
**Weissfrauenstrasse 9**
**W-6000 Frankfurt am Main 1 (DE)**

(72) Erfinder: **Kleemann, Axel, Prof. Dr.**
**Bornweg 36**
**W-6052 Mühlheim 2 (DE)**
Erfinder: **Klenk, Herbert, Dr.**
**Gleiwitzer Strasse 21**
**W-6450 Hanau 9 (DE)**
Erfinder: **Huthmacher, Klaus, Dr.**
**Lärchenweg 18**
**W-6460 Gelnhausen (DE)**
Erfinder: **Most, Dieter, Dr.**
**Fritz-Schubert-Ring 46**
**W-6454 Bruchköbel (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

## Beschreibung

Die Erfindung betrifft ein neues Herstellungsverfahren für m-Trifluormethylphenylacetonitril.

Das m-Trifluormethylphenylacetonitril ist eine Verbindung, die als Ausgangsstoff für die Herstellung einer Vielzahl von Arzneimitteln und von Herbiziden dient. Ihre Herstellung erfolgte bisher beispielsweise in der Weise, daß man m-Trifluormethylbenzol mit Chlormethylether in Gegenwart von Chlorsulfonsäure umsetzte und das Umsetzungsprodukt weiter mit Natriumcyanid behandelte (US-PS 4 144 265). Abgesehen davon, daß man nach diesem Verfahren die gewünschte Verbindung in einer Ausbeute von nur 31%, bezogen auf das Benzoltrifluorid, erhalten konnte, sind mit diesem Verfahren noch folgende Nachteile verbunden :

Der präparative Aufwand ist beträchtlich (Zweistufenprozeß), wobei vor allem die erste Stufe, die Chlormethylierung von Benzotrifluorid (desaktivierter Aromat), größere Schwierigkeiten in sich birgt. So bereitet der Umgang mit den Einsatzstoffen Chlorsulfonsäure und Chlormethylether, insbesondere bei der wäßrigen Aufarbeitung des Rohproduktes, erhebliche Probleme, da zur Entfernung des cancerogenen Chlormethylethers weitere Reinigungsschritte notwendig sind (US-PS 3 994 984). Kritisch ist auch die Frage der Abfallbeseitigung zu beurteilen. Ausbeutelimitierend wirkt sich vor allem die Bildung von Nebenprodukten aus, z.B. mehrfach chlormethylierte Produkte oder die Bildung von Kondensationsprodukten, wie Bis-(3-Trifluormethylphenyl)methan.

Es ist auch bekannt, m-Trifluormethylphenylacetonitril dadurch herzustellen, daß man Trifluormethylbenzol zunächst einer Chlormethylierung unterwirft und dann nach der Kolbeschen Nitrilsynthese mit Natriumcyanid umsetzt (DE-OS 21 50 399). Dieses Verfahren ist jedoch mit folgenden Nachteilen verbunden :

Da es sich um ein Zweistufenverfahren handelt, ist auch hier der präparative Aufwand sehr groß, wobei vor allem die erste Stufe, die Chlormethylierung von Benzotrifluorid (desaktivierter Aromat), größere Probleme mit sich bringt. Bringt man Chlorschwefelsäure in p-Formaldehyd ein (US-PS 3 465 051), so resultiert anfänglich eine zähe, klebrige Masse, die sich nur noch unter Schwierigkeiten rühren läßt und größere technische Probleme aufwirkt. Neben der Aufarbeitung ist vor allem die Nebenproduktbildung kritisch zu betrachten, da nach diesem Verfahren bis zu 30% Bis-(3-Trifluormethylphenyl)methan gebildet wird. Dies hat eine deutliche Minderung der Ausbeute zur Folge.

Es wurde nun gefunden, daß man m-Trifluormethylphenylacetonitril in Ausbeuten über 80%, bezogen auf m-Trifluormethyltoluol, herstellen kann, wenn man m-Trifluormethyltoluol mit Chlorcyan in der Gasphase bei einer Temperatur zwischen 600 und 750°C umsetzt.

Zweckmäßigerweise setzt man hierbei das m-Trifluormethyltoluol und das Chlorcyan in einem Molverhältnis von 3 : 1 ein. Es ist jedoch vorteilhaft, das m-Trifluormethyltoluol in einem Überschuß bis zu 8 : 1 einzusetzen.

Bevorzugt wird das Verfahren bei einer Temperatur von etwa 650°C durchgeführt.

Zweckmäßigerweise geht man bei der Durchführung des erfindungsgemäßen Verfahrens so vor, daß man die beiden Reaktionspartner gasförmig und voneinander getrennt in den Reaktor einspeist. Hierbei sollten beide Reaktionspartner beim Eintritt in den Reaktor bereits nahezu auf die Reaktionstemperatur vorgeheizt sein. Die Aufarbeitung der Reaktionsprodukte kann in an sich bekannter Weise, beispielsweise durch Destillation, erfolgen.

Die Verweilzeiten im Reaktor sollten etwa 10-50 sec betragen.

Es ist zwar aus der DE-PS 28 54 210 bekannt, aromatisch oder heteroaromatisch substituierte Acetonitrile durch Umsetzung von methylsubstituierten Aromaten oder Heteroaromaten mit Chlorcyan in der Gasphase bei Temperaturen etwa zwischen 550 und 850°C durchzuführen, jedoch hat man bisher niemals dieses Verfahren auf m-Trifluormethyltoluol angewendet, weil man Fragmentierungen – insbesondere die Abspaltung von $\cdot CF_3$ und $\cdot CH_3$ und Austauschreaktionen Fluor gegen Chlor/Cyanid befürchten mußte. Eine mögliche HF-Bildung hätte die Zerstörung des Reaktors und des Aufarbeitungsteils zur Folge. Es war demnach durchaus überraschend, daß auf diesem Wege das m-Trifluormethylphenylacetonitril zugänglich ist.

Das als Ausgangsstoff verwendete m-Trifluormethyltoluol kann beispielsweise nach dem in der europäischen Patentschrift 0 084 128 beschriebenen Verfahren hergestellt werden, indem man beispielsweise Benzotrifluorid mit einem Alkylierungsmittel in Gegenwart von Fluorwasserstoff im Temperaturbereich von 0 bis 150°C umsetzt.

Beispiel 1 :

2,11 Mol 3-Trifluormethyltoluol und 0,38 Mol Chlorcyan (Molverhältnis 5,6 : 1) werden durch eine Quarzrohr (Ø 3,6 cm, l 100 cm), das in einem elektrischen Ofen erhitzt wurde, durchgeleitet. Die durchschnittliche Temperatur des Reaktionsrohres betrug 650°C, die Kontaktzeit 12,7 sec. Die heißen Reaktionsgase werden anschließend bei 90 bis 95°C kondensiert, so daß leicht flüchtige Nebenprodukte, wie Chlorwasserstoffgas,

unumgesetztes Chlorcyan u.a., ausgetrieben und der Entgiftung zugeführt werden.

Die gaschromatographisch durchgeführte Analyse des Rohproduktes ergab eine Ausbeute an 3-Trifluormethylphenylacetonitril von

80,8% (bezogen auf 3-Trifluormethyltoluol),
55,3% (bezogen auf Chlorcyan).

Durch eine fraktionierende Destillation läßt sich das Umsetzungsprodukt (Siedepunkt 92 bis 93°C/4 mm) von unumgesetztem 3-Trifluormethyltoluol (Siedepunkt 131 bis 133°C/760 mm) ohne nennenswerte Destillationsverluste abtrennen.

Die Identifizierung wurde spektroskopisch (mit Vergleichssubstanzen) durchgeführt.

Beispiel 2 :

0,40 Mol 3-Trifluormethyltoluol und 0,125 Mol Chlorcyan (Molverhältnis 3,2 : 1) wurden unter gleichen Bedingungen, wie im Beispiel 1 beschrieben, umgesetzt. Die durchschnittliche Temperatur des Reaktorrohres betrug 650°C, die Kontaktzeit 40 sec. Die gaschromatographisch durchgeführte Analyse des Rohrproduktes ergab eine Ausbeute an 3-Trifluormethylphenylacetonitril von

72,7% (bezogen auf 3-Trifluormethyltoluol),
64,0% (bezogen auf Chlorcyan).

Beispiel 3 :

0,40 Mol 3-Trifluormethyltoluol und 0,125 Mol Chlorcyan (Molverhältnis 3,2 : 1) wurden unter den in Beispiel 1 beschriebenen Bedingungen umgesetzt. Die durchschnittliche Temperatur des Reaktorrohres betrug 700°C, die Kontaktzeit 38 sec. Die gaschromatographisch durchgeführte Analyse des Rohrproduktes ergab eine Ausbeute an 3-Trifluormethylphenylacetonitril von

53,3% (bezogen auf 3-Trifluormethyltoluol),
64,0% (bezogen auf Chlorcyan).

Beispiel 4 :

2,11 Mol m-Trifluormethyltoluol und 0,38 Mol Chlorcyan (Molverhältnis 5,6 : 1) wurden unter den in Beispiel 1 beschriebenen Bedingungen umgesetzt. Die durchschnittliche Temperatur des Reaktorrohres betrug 700°C, die Verweilzeit 12,2 sec. Die gaschromatographisch durchgeführte Analyse des Rohproduktes ergab eine Ausbeute an 3-Trifluormethylphenylacetonitril von

70,5% (bezogen auf 3-Trifluormethyltoluol),
81,6% (bezogen auf Chlorcyan).

## Patentansprüche

1. Verfahren zur Herstellung von m-Trifluormethylphenylacetonitril der Formel

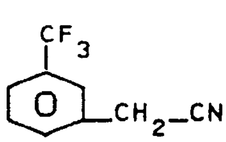 ,

dadurch gekennzeichnet, daß man m-Trifluormethyltoluol mit Chlorcyan in der Gasphase bei einer Temperatur

zwischen 600° und 750°C umsetzt.

2. Verfahren nach Anspruch 1, dadurch gekennzeichnet, daß man das m-Trifluormethyltoluol und Chlorcyan in einem Molverhältnis von 3 : 1 einsetzt.

3. Verfahren nach den Ansprüchen 1 und 2, dadurch gekennzeichnet, daß man das m-Trifluormethyltoluol in einem Überschuß bis zu 8 : 1 einsetzt.

4. Verfahren nach den Ansprüchen 1 bis 3, dadurch gekennzeichnet, daß man bei einer Temperatur von etwa 650°C umsetzt.

## Revendications

1. Procédé pour la préparation de méta-trifluorométhylphénylacétonitrile de la formule :

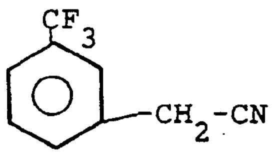

caractérisé en ce que l'on transforme du métatrifluorométhyltoluène avec du chlorcyan en phase gazeuse à une température comprise entre 600 et 750°C.

2. Procédé selon la revendication 1, caractérisé en ce que l'on introduit le méta-trifluorométhyltoluène et le chlorcyan dans un rapport molaire de 3 à 1.

3. Procédé selon l'une quelconque des revendications 1 et 2, caractérisé en ce que l'on introduit le méta-trifluorométhyltoluène en excès, dans un rapport pouvant aller jusqu'à 8 à 1.

4. Procédé selon l'une quelconque des revendications 1 à 3, caractérisé en ce que l'on fait la réaction à une température d'environ 650°C.

## Claims

1. A process for the production of m-trifluoromethyl phenyl acetonitrile corresponding to the formula

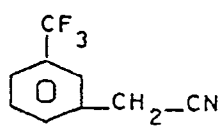

characterized in that m-trifluoromethyl toluene is reacted with cyanogen chloride in the gas phase at a temperature of 600 to 750°C.

2. A process as claimed in claim 1, characterized in that m-trifluoromethyl toluene and cyanogen chloride are used in a molar ratio of 3 : 1.

3. A process as claimed in claims 1 and 2, characterized in that the m-trifluoromethyl toluene is used in an excess of up to 8 : 1.

4. A process as claimed in claims 1 to 3, characterized in that the reaction is carried out at a temperature of approximately 650°C.